# FASCICULE DE BREVET EUROPEEN

(11) **EP 1 017 715 B1**
(45) Date de publication et mention de la délivrance du brevet: **28.05.2008**
(21) Numéro de dépôt: 98939705.4
(22) Date de dépôt: 17.07.1998
(51) Int. Cl.: C12N 15/11, C07K 14/25, C07K 14/245, C07K 14/47, C07K 14/705, A61K 38/04, C12N 15/62

(54) **POLYPEPTIDE CHIMERIQUE COMPRENANT LE FRAGMENT B DE LA TOXINE SHIGA ET DES PEPTIDES D'INTERET THERAPEUTIQUE**
CHIMÄRE POLYPEPTIDE, DIE DAS FRAGMENT B DES SHIGATOXINS SOWIE PEPTIDE VON THERAPEUTISCHEM INTERESSE ENTHALTEN
CHIMERIC POLYPEPTIDE COMPRISING THE FRAGMENT B OF SHIGA TOXIN AND PEPTIDES OF THERAPEUTIC INTEREST

(30) Priorité: 18.07.1997 FR 9709185
(43) Date de publication de la demande: 12.07.2000
(73) Titulaire: Institut Curie, 75248 Paris Cedex 05 (FR); CENTRE NATIONAL DE LA RECHERCHE SCIENTIFIQUE, 75016 Paris Cédex (FR)
(72) Inventeur: GOUD, Bruno, F-75015 Paris (FR); JOHANNES, Ludger, F-75020 Paris (FR)
(74) Mandataire: Desaix, Anne
(86) Numéro de dépôt international: PCT/FR1998/001573
(87) Numéro de publication internationale: WO 1999/003881

(56) Documents cités:
- EP-A- 0 439 954
- WO-A-93/16186
- WO-A-93/17115
- WO-A-97/13410
- A RAPAK ET AL.: "Retrograde transport of mutant ricin to the endoplasmic reticulum with subsequent translocation to the cytosol" PROCEEDINGS OF THE NATIONAL ACADEMY OF SCIENCES OF USA., vol. 94, avril 1997, pages 3783-3788, XP002061694 WASHINGTON US
- L JOHANNES & B GOUD: "Shiga toxin as a tool to study retrograde transport" ANNUAL MEETING OF THE 6TH INTERNATIONAL CONGRESS ON CELL BIOLOGY AND THE 36TH AMERICAN SOCIETY FOR CELL BIOLOGY, SAN FRANCISCO, CALIFORNIA, USA, DECEMBER 7-11, 1996. MOLECULAR BIOLOGY OF THE CELL, vol. 7, no. suppl., 1996, page 75a XP002061695
- L JOHANNES ET AL.: "Retrograde transport of KDEL-bearing B-fragment of Shiga toxin" JOURNAL OF BIOLOGICAL CHEMISTRY., vol. 272, no. 31, 1 août 1997, pages 19554-19561, XP002061696 MD US
- BIOCHEMISTRY., vol. 36, 1997, pages 11051-11054, US AMERICAN CHEMICAL SOCIETY. EASTON, PA.
- JOURNAL OF INFECTIOUS DISEASES., vol. 171, 1995, pages 721-724, US CHICAGO, IL.
- INFECTION AND IMMUNITY., vol. 55, 1987, pages 298-303, US AMERICAN SOCIETY FOR MICROBIOLOGY. WASHINGTON.

### LISTAGE DE SEQUENCE

<110> INSTITUT CURIE
   CENTRE NATIONAL DE LA RECHERCHE SCIENTIFIQUE
<120> POLYPEPTIDE CHIMERIQUE COMPRENANT LE FRAGMENT B DE LA TOXINE SHIGA ET DES PEPTIDES D'INTERET THERAPEUTIQUE
<130> 3509A/FL/SDU
<140> PCT/FR98/01573
   <141> 1998-07-17
<150> 9709185
   <151> 1997-07-18
<160> 7
<170> PatentIn Vers. 2.0
<210> 1
   <211> 49
   <212> ADN
   <213> Artificial Sequence
<220>
   <223> Description de séquence Artificiel : amorce PCR
<400> 1
   actagctctg aaaaggatga actttgagaa ttctgactca gaatagctc 49
<210> 2
   <211> 56
   <212> ADN
   <213> Artificial Séquence
<220>
   <223> Description de Séquence Artificiel : amorce PCR
<400> 2
   cttttcagag ctagtagaat taggatgata gcggccgcta cgaaaaataa cttcgc 56
<210> 3
   <211> 17
   <212> ADN
   <213> Artificial Sequence
<220>
   <223> Description de sequence Artificiel : amorce sp,cifique au vecteur ShigaAtpE
<400> 3
   cactactacg ttttaac 17
<210> 4
   <211> 15
   <212> ADN
   <213> Artificial Sequence
<220>
   <223> Description de Séquence Artificiel: amorce sp,cifique au vecteur shigaAtpE
<400> 4
   cggcgcaact atcgg 15
<210> 5
   <211> 24
   <212> ADN
   <213> Artificial sequence
<220>
   <223> Description de Séquence Artificiel : fragment adaptateur contenant le site de glycosylation
<400> 5
   ggccgccatc ctaattctac ttct 24
<210> 6
   <211> 23
   <212> ADN
   <213> Artificial Sequence
<220>
   <223> Description de sequence Artificiel : fragment adaptateur contenant le site de glycosylation
<400> 6
   ctcagaagta gaattaggat ggc 23
<210> 7
   <211> 28
   <212> ADN
   <213> Artificial Séquence
<220>
   <223> Description de séquence Artificiel : fragment adaptateur contenant le site de glycosylation
<400> 7
   gagtctgaaa aagatgaact ttgatgag 28

## Description

L'invention a pour objet des moyens et leur utilisation pour le transport intracellulaire des protéines ou polypeptides ainsi que la présentation membranaire de certains épitopes.

Le transport rétrograde peut être défini comme le cheminement des molécules de la membrane cellulaire vers le réticulum endoplasmique (RE), en passant le cas échéant par l'appareil de Golgi. Ce mécanisme a été mis en évidence pour certaines classes de protéines du réticulum endoplasmique porteuses du tétrapeptide KDEL à leur extrémité carboxy-terminale (ou HDEL dans la levure). Tant des évidences biochimiques que morphologiques indiquent que ces protéines quittent le réticulum endoplasmique, atteignent l'appareil de Golgi dans lequel elles subissent des modifications dans leur chaîne carbohydrate puis sont redirigées vers le réticulum endoplasmique. Le tétrapeptide KDEL est un signal de rétention qui permet de piéger le peptide ou la protéine à laquelle il est attaché dans le réticulum endoplasmique, ce piégeage ayant lieu par interaction à une protéine récepteur de ce motif KDEL décrit par Lewis M.J. et al dans Nature, 348 (6297) : 162 : 3, 1990 November 8.

D'autres évidences sur l'existence d'un transport rétrograde intracellulaire viennent de l'étude de certaines toxines bactériennes qui entrent dans le cytosol des cellules eucaryotes après passage dans le réticulum endoplasmique (Pelham et al. (1992) Trends cell. Biol., 2 : 183-185). Un exemple particulièrement étudié est celui de la toxine de Shiga de Shigella dysenteriae, ainsi que les toxines de type Shiga de E. coli. Ces toxines sont composées de deux chaînes polypeptidiques, l'une (le fragment A) est le fragment toxique et porte une activité déadenylase qui inhibe la synthèse protéique par action sur l'ARN ribosomal 28S, alors que l'autre sous-unité (fragment B) permet le couplage de la toxine sur sa cible (O'Brien et al (1992), Curr. Top. Microbiol. Immunol. 180: 65-94). Des études en microscopie électronique ont montré que la toxine de Shiga peut être détectée dans le RE des cellules A 431, Vero, des cellules Daudi notamment (Sandvig et al,1992 et 1994; KHINE, 1994). En outre, le traitement des cellules avec un métabolique fongique qui provoque la perte de la structure de l'appareil de Golgi (brefeldine A) protège les cellules contre la toxine de Shiga suggérant ainsi qu'elles traversent l'appareil de Golgi avant d'atteindre le RE. Kim et al (1996) ont enfin confirmé que le fragment B de la toxine est localisée dans l'appareil de Golgi.

L'état des connaissances sur le transport rétrograde, et notamment le transport du fragment B de la toxine Shiga dans le RE est donné dans les références suivantes : Sandvig et al (1992) Nature 358 :510-512 ; Sandvig et al (1994) J. Cell. Biol 126 :53-64 ; Kim et al,(1996) J. Cell. Biol 134 :1387-1399.

Le transport intracellulaire est défini comme l'ensemble des échanges entre les différents compartiments cellulaires.

Les auteurs de la présente demande ont observé que le fragment B était acheminé non seulement vers le RE, mais aussi dans le noyau de cellules des lignées hématopoïétiques notamment les cellules dendritiques et les macrophages.

Les auteurs ont montré que ces cellules, incubées en présence de deux micro-molaires du fragment B-gly-KDEL tel que décrit ci-dessous pendant trois heures puis fixées, présentaient une réactivité avec des anticorps spécifiques contre la toxine dans le noyau et même dans le nucléole de ces cellules (résultats non publiés), ce qui indique bien l'existence d'un transport intracellulaire dudit fragment.

La présente invention résulte des observations sur le transport intracellulaire du fragment B de la toxine de Shiga (le Fragment B) et utilise ses propriétés d'acheminement pour construire une séquence polypeptidique chimérique contenant :
- soit un peptide ou polypeptide d'intérêt thérapeutique lié à ce fragment ou tout équivalent fonctionnel de celui-ci.
- soit une séquence polynucléotide portant une séquence dont l'expression est recherchée. Le couplage entre le fragment B et la séquence polynucléotidique est réalisé par toute technique connue de l'homme du métier et notamment celle décrite par Allinquant B. et al dans Journal of Cell Biology, 1288 (5) : 919-27, (1995).

Outre le couplage covalent de molécules d'ADN ou d'autres molécules au fragment B, un couplage peut être réalisé par l'intermédiaire d'une interaction non covalente forte. Pour cela, et à titre d'exemple, l'ADN_{c} du fragment B est fusionné avec celui de la streptavidine ou avec tout autre dérivé de l'avidine, selon les méthodes décrites (Johannes et al. (1987) J. Biol. Chem., 272: 19554-19561).

La protéine résultant de la fusion (B-Streptavidine) peut être soumise à réaction avec l'ADN biotinylé obtenu par PCR en utilisant des amorces biotinylées, ou avec tout autre substance biotinylée. Le complexe résultant est lié aux cellules cibles et devrait être transporté comme le fragment B intracellulaire.

Selon une autre méthode de couplage, on a recours à une étape de biotinylation site-spécifique du fragment B. Pour cela, l'ADN_{c} du fragment B est fusionné avec l'ADN_{c} codant pour le site de reconnaissance de l'enzyme BirA (Boer et al. (1995) J. Bacterial, 177: 2572-2575 ; Saou et al. (1996) Gene, 169: 59-64). Après biotinylation in vitro, le fragment B est lié à d'autres molécules biotinylées (telles que des ADN_{c}, voir ci-dessus) par l'intermédiaire de la streptavidine ou de tout autre dérivé tétravalent de l'avidine.

Par équivalent fonctionnel, on entend toute séquence dérivée du Fragment B par mutation, délétion ou addition, et présentant les mêmes propriétés d'acheminement que le Fragment B .

De façon élargie, un équivalent fonctionnel peut être constitué par tout fragment présentant les propriétés de transport rétrograde et même de transport intracellulaire jusqu'au noyau, que celles décrites pour le Fragment B. A titre d'exemple, on peut citer le Fragment B de la verotoxine décrit dans Proceedings of the National Academy of Sciences of the United States of America, 84 (13):4364-8; 1987-July, ou le Fragment B de la ricine décrit par Lamb F.I. et al dans European Journal of Biochemistry, 148 (2) : 265-70, (1995). Après description des propriétés particulières de transport de ces fragments, l'homme du métier saura choisir le fragment qui sera le meilleur candidat comme vecteur d'acheminement d'une séquence quelconque dans un compartiment cellulaire quelconque.

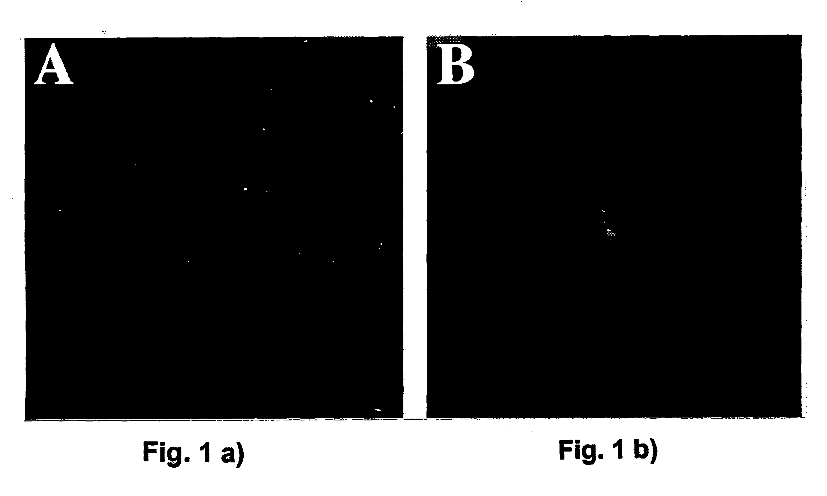

Ainsi, la présente demande couvre l'utilisation du fragment B de la toxine de Shiga, ou tout autre sous-unité de toxines bactériennes qui aurait des activités comparables, et en particulier des propriétés d'acheminement analogues à celles du fragment B, y compris des polypeptides mimant le fragment B de la toxine de Shiga. Ces polypeptides, et de façon générale ces équivalents fonctionnels peuvent être identifiés par des méthodes de criblage (Screening methods) qui ont en commun le principe de la détection de l'interaction entre des séquences peptidiques aléatoires (random) et le récepteur Gb₃, ou des analogues solubles de ce récepteur. A titre d'exemple, l'utilisation de banques de phages (phage-library) exprimant des séquences peptidiques aléatoires pour la sélection sur des colonnes d'affinité comportant du Gb₃ ou après hybridation à des analogues solubles et radioactifs du Gb₃ peut être mise en oeuvre. Le glycolipide Gb₃ a été identifié comme étant le récepteur cellulaire de la toxine de Shiga (Lingwood (1993), Adv. Lipid Res., 25: 189-211). Gb₃ est exprimé par les cellules sensibles à la toxine et l'internalisation de la toxine serait permise par le biais d'une interaction avec Gb₃. Les inventeurs de la présente demande ont montré que dans des cellules HeLa dans lesquelles l'expression du récepteur Gb₃ a été inhibée (figure 1)A), le fragment B internalisé n'est pas transporté dans l'appareil de Golgi mais est accumulé au niveau des structures vésiculaires dans le cytoplasme, principalement représentées par les lysosomes. Dans les cellules contrôle, le fragment B est transporté au niveau de l'appareil de Golgi (figure 1)B).

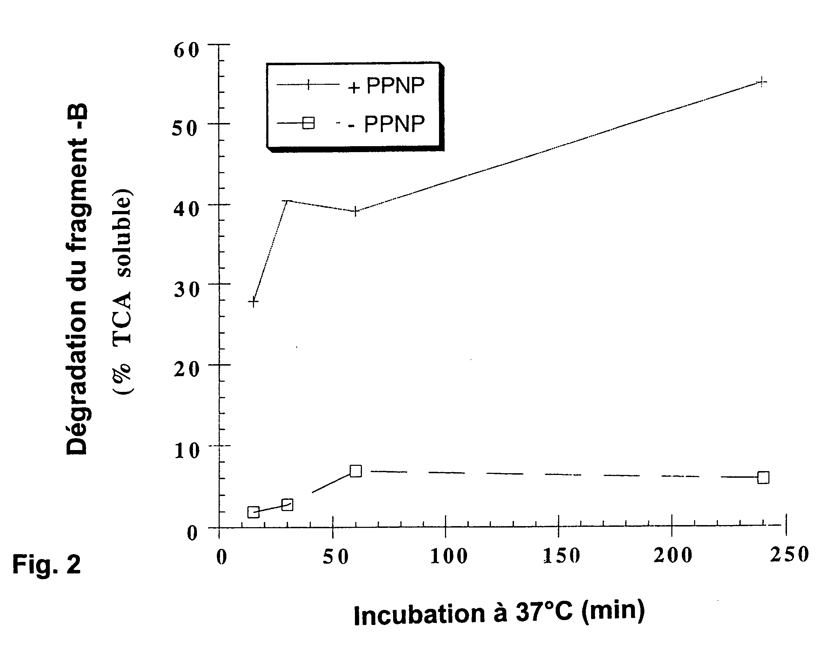

Cette hypothèse selon laquelle en l'absence du récepteur Gb₃, le fragment B n'est plus transporté vers le système de biosynthèse ou de sécrétion, est confirmée par des expériences biochimiques (figure 2).

Les inventeurs ont montré qu'en présence de l'inhibteur de synthèse du récepteur Gb₃, PPNP (+PPNP), jusqu'à 50 % du fragment B internalisé est dégradé sous forme de matériel TCA soluble, ce qui est conforme à une activité de transport vers un compartiment de dégradation ultérieure tel que un compartiment endosomal ou lysosomal. Lorsque la synthèse du récepteur Gb₃ n'est pas inhibée (-PPNP), une proportion beaucoup plus faible de fragment B internalisé devient TCA soluble. On peut donc conclure que la présence du récepteur Gb₃ est nécessaire pour l'adressage du fragment B dans des compartiments spécifiques, ce qui plaiderait en faveur du fait que l'aspect prépondérant de l'activité du fragment B serait sa liaison au récepteur Gb₃.

La présente invention a pour objet des séquences polypeptidiques chimériques, lesdites séquences comprenant au moins : le fragment B de la toxine de Shiga ou un équivalent fonctionnel de celui-ci à laquelle est liée à l'extrémité carboxy-terminale un ou plusieurs polypeptides X, répondant à la formule suivante :

B - X,

dans laquelle :
- B représente le fragment B d'une toxine comme celle de Shiga, dont la séquence est décrite dans N. G. Seidah et al, (1986) J. Biol. Chem. 261 : 13928-31, et dans Strockbine et al (1988) J. Bact. 170 : 1116-22, ou un équivalent fonctionnel de celle-ci, ou de verotoxine ou de ricine (références supra).
- X représente un ou plusieurs polypeptides dont la longueur totale a comme limite supérieure celle de la compatibilité avec un transport rétrograde ou intracellulaire.

La présente invention porte également sur des molécules chimériques de structure

B - X'

dans lesquelles B à la même signification que ci-dessus et X' représente une séquence nucléotidique codant une séquence peptidique X dont l'expression est recherchée, notamment un épitope d'antigène.

Les molécules chimériques de l'invention peuvent en outre comporter :
a) des sites de modification, tels un site de N- glycosylation constitué d'environ 20 acides aminés, des sites de phosphorylation ou toute séquence nécessaire à une éventuelle maturation de la molécule.
b) un signal de rétention de type du tétrapeptide KDEL(Lys-Asp-Glu-Leu) qui, quand il est lié à l'extrémité carboxy-terminale des protéines résidentes du RE, entraîne une rétention après la maturation des protéines par passage dans l'appareil de Golgi. Une synthèse sur le rôle du signal de rétention dans la maturation protéique est proposée dans M. J. Lewis et al, (1992) Cell, 68 :353-64.

De façon plus générale, les séquences polypeptidiques chimériques pourront comprendre :
- toute séquence nécessaire à la maturation de la protéine dans un système cellulaire adapté ;
- toute séquence nécessaire à la reconnaissance d'un type cellulaire donné par la molécule chimérique, permettant ainsi une sélectivité d'action et de pénétration dans le cytoplasme de la cellule.

Le point commun à toutes les séquences de structure B - X ou B - X' chimériques est qu'elles contiennent le fragment B, ou un équivalent fonctionnel de celui-ci.

Les molécules chimériques de l'invention permettent un acheminement des séquences X ou du produit d'expression de X' dans le RE. Quand X est lié au Fragment B, le transport rétrograde passe également par l'appareil de Golgi et probablement par les endosomes. En outre, dans certaines conditions, les molécules de l'invention peuvent subir une maturation conduisant à une présentation membranaire de certains épitopes contenus dans la séquence polypeptidique chimérique.

Par maturation, on entend tout processus qui, à partir d'un polypeptide donné, conduit à l'émergence de peptides qui, eux-mêmes, pourront être présentés dans un compartiment cellulaire y compris le cytoplasme. La maturation peut être effectuée, soit par coupure enzymatique dans le réticulum endoplasmique, soit par transport dans le cytoplasme dans lequel le polypeptide subit un clivage puis les peptides ainsi obtenus sont transportés à nouveau dans le réticulum endoplasmique.

Les molécules du complexe majeur d'histocompatibilité de classe I (CMH cl I) peuvent se charger des molécules polypeptidiques d'intérêt X ou X' après un tel clivage et être présentées sur les membranes cellulaires.

Quand la molécule chimérique de l'invention consiste en un couplage d'un fragment B de son équivalent avec une molécule polynucléoditique ou un vecteur d'expression comportant une séquence dont l'expression est recherchée, le polypeptide ainsi synthétisé, après transcription dans le noyau puis traduction dans le cytoplasme, pourra subir les mêmes étapes de clivage, maturation et transport intracellulaire que ce qui est décrit ci-dessus pour une séquence chimérique polypeptidique.

Des molécules polypeptidiques chimériques conformes à l'invention peuvent constituer un principe actif dans une composition thérapeutique pour l'immunothérapie par un mécanisme qui se rapproche des processus biologiques quant à la présentation antigénique appropriée au développement de la réaction immune. Le fragment X représente alors un ou plusieurs épitopes dont la présentation membranaire est recherchée à la surface des cellules. La taille du fragment X est limitée uniquement par la capacité de transit intracellulaire des molécules chimériques concernées.

Cette approche peut être envisagée tant pour une immunothérapie anti-infectieuse ou anti-cancéreuse que pour constituer un leurre antigénique dans certaines maladies auto-immunes.

Tout type d'antigènes présentés par des CMH cl I est un bon candidat pour sélectionner des épitopes simples ou chimériques qui font partie des constructions de l'invention. A titre d'exemple nous citerons :

### a) Epitopes humains dérivés de protéines de cellules de mélanome :

- BAGE issu de la Tyrosinase (Boel, P. et al (1995), Immunity 2, 167-75) ;
- GAGE issu de la gp75 (Van den Eynde, B. et al (1995), J. Exp. Med. 182, 689-98) ;
- Tyrosinase (Brichard V. et al (1993), J. Exp. Med. 178, 489-95)
- p15 issu de la Melan A/MART-1 (Coulie P.G. et al (1994), J. Exp. Med. 180, 35-42 ; Kawakami Y. et al. (1994), J. Exp. Med. 180, 347-52) ;
- MAGE-1 et -3 issus de la β-caténine (De Plaen E. et al (1994), Immunogenetics 40, 369-9 ; Traversari C et al (1992), J. Exp. Med. 176, 1453-7).

### b) Epitopes humains dérivés de protéines de virus impliqués dans le développement du cancer :

- Peptides dérivés des protéines E6 et E7 du HPV 16 (Feltkamp M.C. et al (1993), Eur. J. Immunol. 23, 2242-9 ; Davis H.L. et al (1995), Hum Gene Ther 6, 1447-56) ;
- Peptides dérivés de la protéine Hbs du HBV (Rehermann B. et al (1995), J. Exp. Med. 181, 1047-58) ;
- Peptides dérivés des protéines du EBV (Murray R.J. et al (1992), J. Exp. Med. 176, 157-68) ;
- Peptide dérivé du cytomégalovirus

### c) Epitopes humains dérivés d'oncogènes :

- p21 ras (Peace D.J. (1993), J. Immumnother 14, 110-4 ; Ciernik, I.F. et al (1995) Hybridoma 14, 139-42) ;
- p53 (Gnjatic S. (1995), Eur. J. Immunol. 25, 1638-42)

### d) Epitopes présentant un intérêt pour les maladies autoimmunes :

Ces épitopes peuvent être choisis parmi ceux décrits par Chiez R.M. et al (1994) dans Immunol. Today 15,155-60.

### e) Epitopes présentant un intérêt dans le contexte des maladies infectieuses :

A titre d'exemple de tels épitopes, on peut citer ceux décrits par Furukawa K. et al (1994) dans J. Clin. Invest. 94, 1830-9.

Dans les constructions de l'invention, X ou le produit d'expression de X' peut également représenter une séquence polypeptidique permettant la restauration d'une fonction du transport intracellulaire perturbée par quelque cause que ce soit. A titre d'exemple, une molécule biologique peut être piégée dans le RE du fait d'une modification par mutation, délétion ou addition d'une séquence, ayant pour effet de bloquer la maturation ou le transit de cette molécule. C'est le cas, par exemple, du mutant CFTR (Δ F508) dont la liaison à une molécule chaperonne, telle la calnexine, est modifiée de telle manière que son relargage est empêché ou retardé, empêchant ainsi le transit intracellulaire. Cette mutation est la cause de la mucoviscidose. L'introduction dans le réticulum endoplasmique d'une réplique non mutée pourrait permettre de déplacer les chaîne N-glycosylées de la glycoprotéine CFTR (Δ F508) du site d'interaction avec la calnexine, ayant pour effet de permettre à nouveau le transport de la protéine jusqu'à la membrane plasmique, et un fonctionnement normal des cellules épithéliales du poumon.

L'invention porte également sur des constructions d'acides nucléiques , et en particulier d'ADN ou d'ADNc comprenant une séquence de nucléotides codant pour la protéine chimérique dont la structure et ses différentes variantes sont définies ci-dessus. Plus particulièrement, l'invention porte sur des vecteurs d'expression ou plasmides porteurs des constructions ci-dessus et susceptible de les exprimer dans des cultures bactériennes. A titre d'exemple, le vecteur d'expression peut être le plasmide pSU108 décrit dans G. F. Su et al, (1992) Infect. Immun., 60 :3345-59.

Plus particulièrement, l'invention porte sur des constructions comprenant :
- la séquence codant pour le fragment B,
- une séquence codant pour un ou plusieurs polypeptides dont l'expression est recherchée. Il peut s'agir d'épitopes dont l'expression membranaire est recherchée à la surface des cellules ; il peut s'agir également de polypeptides permettant la rétention des protéines dans l'appareil de Golgi ; il peut s'agir enfin de polypeptides permettant de restaurer un fonctionnement perturbé du transport intracellulaire.

La construction pourra en outre comporter, toute séquence d'acide nucléique codant pour un polypeptide dont la présence permet un transport intracellulaire correct dans les cellules destinées à être traitées par les molécules de l'invention. Il pourra s'agir en particulier :
d'une séquence codant pour un signal de N-glycosylation,
- d'une séquence codant pour le signal de rétention KDEL.

La construction polynucléotidique de l'invention est sous le contrôle d'un promoteur, de préférence un promoteur fort permettant un taux d'expression correct dans les bactéries dans lesquelles il a été transfecté.

L'invention a également pour objet des bactéries transfectées comprenant ces constructions, et susceptibles de produire les protéines ou polypeptides chimériques de l'invention.

Les cellules hôtes traitées par les molécules de l'invention font également partie de l'invention ; elles peuvent être tout type de cellules et notamment :
- celles qui peuvent être traitées *ex vivo* comme les cellules du système immunitaire actives dans le déclenchement de l'immunité cellulaire, telles les cellules dendritiques, les macrophages, ou les lymphocytes B ;
- celles qui peuvent être traitées *in situ* comme les cellules epithéliales utilisables dans la restauration de fonctions altérées soit par un déficit génétique, soit par une perturbation métabolique ;
- des cellules cancéreuses.

De manière générale, Les molécules chimériques de l'invention permettent d'approcher une méthode thérapeutique nouvelle qui s'affranchit des problèmes liés aux vecteurs viraux ou rétroviraux habituellement utilisés pour intégrer et faire exprimer des molécules exogènes dans des cellules animales. La méthode thérapeutique, telle qu'elle découle des molécules de l'invention, consiste à traiter directement les cellules d'un patient, soit ex vivo, soit par application directe de type stéréotaxique des séquences polypeptidiques chimériques, ou enfin par les méthodes classiques de traitement mucosal, comme les aérosols.

L'invention porte sur l'utilisation des polypeptides chimériques ou des séquences polynucléotidiques codant pour les polypeptides de l'invention dans la fabrication de compositions thérapeutiques, dans laquelle des polypeptides particuliers sont exprimés au niveau des membranes des cellules cibles. Ces polypeptides sont de façon avantageuse des épitopes contre lesquels le développement d'une réaction immunologique est recherchée qui sont alors présentées à la surface des cellules du système immunitaire, notamment des cellules dendritiques, des macrophages ou des lymphocytes B. Le fragment B de la toxine de Shiga agit comme un vecteur d'épitope permettant de programmer des cellules présentatrices d'antigènes.

La présente invention porte sur une méthode d'immunothérapie, consistant à augmenter l'immunité cellulaire suite à une présence d'un antigène indésirable dans un organisme, ladite méthode consistant à faire exprimer par les cellules clefs de l'immunité cellulaire, comme les cellules dendritiques et les macrophages, des épitopes particuliers. La méthode de traitement selon l'invention vise à déclencher l'immunité à médiation cellulaire et humorale en chargeant les molécules du CMH cl I ou cl II avec les épitopes d'intérêt, après restriction dans les cellules cibles. Cela conduit à une activation des cellules T cytotoxiques à l'encontre de l'antigène dont l'élimination est recherchée.

Les épitopes présentés grâce aux constructions de l'invention sont issus d'antigènes viraux, parasitaires, bactériens, ou de toute cellule, organite, ou micro-organisme dont l'élimination est recherchée, comme peuvent l'être des cellules cancéreuses ou infectées. Les épitopes peuvent également être des leurres permettant aux molécules du « soi » reconnues comme des antigènes étrangers dans des maladies auto-immunes d'être remplacées par les épitopes de l'invention, entraînant ainsi à un ralentissement ou à la diminution de la réaction immunitaire.

Des exemples de ces épitopes sont cités ci-dessus à l'occasion de la description des séquences polypeptidiques chimériques.

L'invention porte également sur l'utilisation des molécules chimériques de l'invention dans la fabrication de compositions thérapeutiques, dans laquelle les polypeptides particuliers que l'on souhaite exprimer permettent la restauration du transit intracellulaire d'une protéine dont la structure altérée conduit à son piégeage dans le RE et à un déficit d'expression. C'est le cas des protéines d'expression membranaire qui subissent une maturation intracellulaire, incluant des glycosylations, sulfations, repliements (folding) etc.

Un exemple particulier est celui du mutant CFTR (Δ F508) dont l'attachement à une molécule chaperonne comme la calnexine est modifié suite à une modification de la protéine ; ce qui conduit à un piégeage de la molécule, cause de la mucoviscidose, et qui se traduit par une insuffisance générale des sécrétions exocrines, notamment au niveau du pancréas et des poumons.

La présente invention porte sur une méthode de traitement thérapeutique de maladies dont l'origine est un défaut de sécrétion de protéines ; la méthode consiste à administrer directement les polypeptides chimériques ou à administrer dans les cellules des patients l'information génétique sous forme de plasmides porteurs de séquences exogènes codant pour un peptide ou polypeptide qui permettra la restauration de la fonction cellulaire déficiente.

Cette restauration peut résulter soit de la supplémentation de la fonction déficiente par le polypeptide X, soit d'une compétition entre la protéine mutée et le polypeptide synthétisé à partir de la séquence exogène pour la liaison avec une molécule ou un récepteur spécifique de la machinerie cellulaire. Un exemple particulier est le traitement du mutant précité, cause de la mucoviscidose, par administration d'un vecteur porteur d'une séquence codant pour le site d'attachement de la protéine CFTR avec sa molécule chaperonne, ou par administration directe du polypeptide chimérique.

Les constructions de l'invention mettent à la disposition de la communauté de la santé humaine ou animale un moyen thérapeutique nouveau pour traiter les maladies ayant pour cause un déficit du transit intracellulaire, ou pour augmenter ou induire une présentation membranaire d'une molécule, d'un polypeptide ou d'un épitope d'intérêt.

D'autres propriétés de l'invention apparaîtront à la lumière des exemples et des figures qui suivent.

### LEGENDE DES FIGURES :

Figure 1)A. : Cellules Hela dans lesquelles l'expression du récepteur Gb₃ a été inhibée. Le fragment B internalisé n'est pas transporté vers l'appareil de Golgi mais est accumulé dans les structures vésiculaires.
Figure 1)B : Cellules Hela contrôles dans lesquelles le fragment B et transporté vers l'appareil de Golgi.
Figurer 2 : Test biochimique montrant le défaut de transport du fragment B.

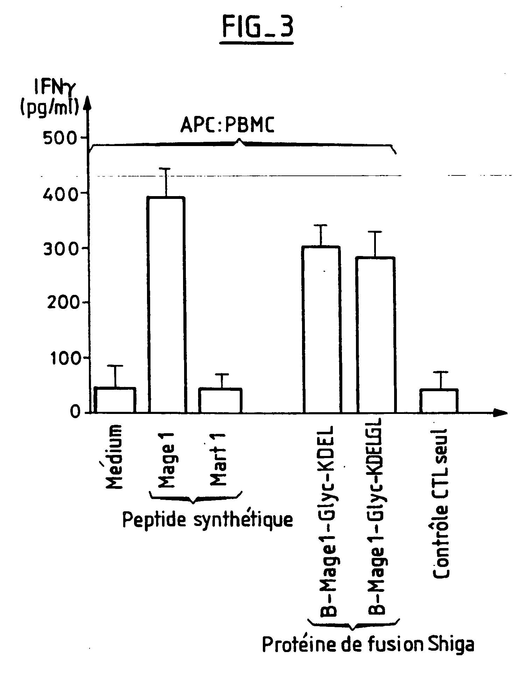

Figure 3 : Présentation CMH classe restreinte de protéines de fusion Shiga B-Mage 1 à des cellules monocytaires du sang périphérique (CMSP ou PBMC) : rôle de la séquence KDEL. Les CMSP (5 x 10⁴) ont été primées pendant une nuit avec soit le peptide Mage 1 (1 µM), soit Mart 1 (1 µM) ou les protéines de fusion Shiga B-Mage 1 (1 µM) avec une séquence de recyclage vers le réticulum endoplasmique active (B-Mage 1-Glyc-KDEL) ou inactive (B-Mage 1-Glyc-KDELGL). Après lavage, 2 x 10⁴ cellules T cytotoxiques spécifiques pour l'épitope Mage 1 (clone 82/30) ont été incubées avec les cellules CMSP primées pendant 24 heures. Les surnageants ont été ensuite collectés et testés pour la production d'interféron γ.

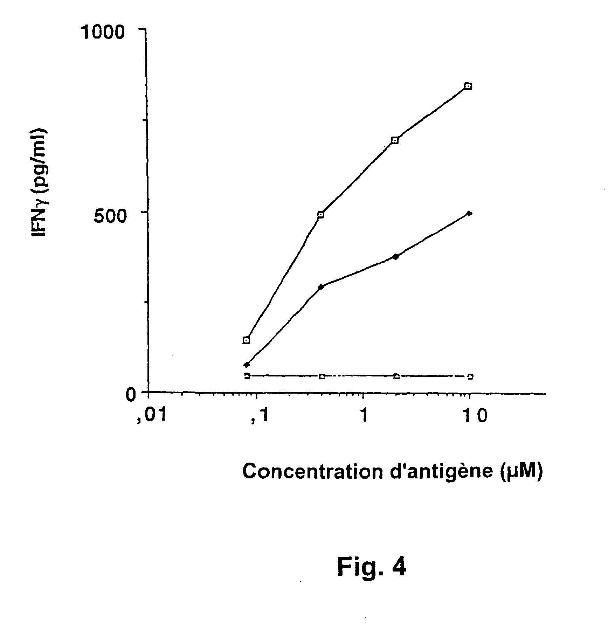

Figure 4 : Présentation CMH classe 1 restreinte de la protéine de fusion Shiga B-Mage 1 par différents types de cellules présentatrices d'antigènes. Des cellules lymphoblastoïdes B (E), des cellules dentritiques (+), ou des cellules T (□) clonales ont été primées avec la protéine de fusion soluble Shiga B-Mage 1 comme dans la figure 3. La présentation de peptides Mage 1 a été testée avec la lignée 82/30 CTL.

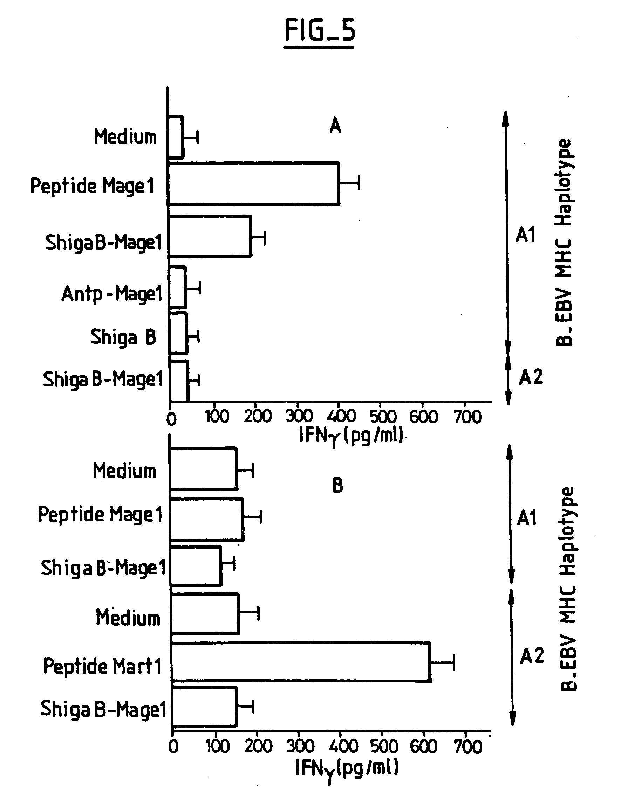

Figure 5 : Analyse de la spécificité de la présentation CMH classe I restreinte de la protéine de fusion Shiga B-Mage 1 par des lignées de cellules lymphoblastoïdes B. Les cellules de la lignée B lymphoblastoïde BM21 (HLA-A1) ou BV1 (HLA-A2) ont été primées pendant-la nuit avec soit le milieu seul, soit les peptides synthétiques Mage 1 ou Mart 1 (1 µM), soit la protéine de fusion Shiga B-Mage 1 (1 µM), soit la protéine de fusion Antp-Mage 1, ou le fragment B de la toxine de Shiga sauvage. Après lavage, les cellules spécifiques de Mage 1 CTL 82/30 (A) ou de Mart 1 CTL LB373 (B) ont été incubées pendant 24 heures avec des cellules B-EBV primées. Les surnageants ont été ensuite collectés et testés pour la production d'interféron γ.

### I - Construction d'un polynucléotide chimérique recombinant et production du polypeptide correspondant.

### I - 1 ) Construction du plasmide.

L'épitope X choisi est l'épitope MAGE, présent dans les cellules cancéreuses de patients atteints de mélanome. Le plasmide utilisé est le plasmide pSU108 décrit dans Su et al., 1992, Infect. Immun. 60:33-45, 3359.

Les amorces PCR utilisés sont les suivantes:
5'-ACTAGCTCTGAAAAGGATGAACTTTGAGAATTCTGACTCAGAATAGCTC-3'

5'-CTTTTCAGAGCTAGTAGAATTAGGATGATAGCGGCCGCTACGAAAA
ATAACTTCGC-3'

Ces amorces sont utilisées avec des amorces spécifiques du vecteur ShigaAtpE (5')
5'-CACTACTACGTTTTAAC-3'
5'-CGGCGCAACTATCGG-3',
afin de produire des fragments qui ont été clonés au niveau des sites de restriction Sphl et SalI du plasmide SU108.

Des fragments adaptateurs contenant le site de glycosylation et la séquence KDEL, composé des oligonucléotides sulfate 1 : ((5' phosphorylated; 5'-GGCCGCCATCCTAATTCTACTTCT-3') et sulfate 2 (5'-CTCAGAAGTAGAATTAGGATGGC-3') ou de sulfate 3 (5'-GAGTCTGAAAAAGATGAACTTTGATGAG-3') ont été ligaturés pendant la nuit à 16°C.

Les fragments résultants ont été clonés dans les sites de restriction de pSU108 Notl et EcoRI et contenant le cDNA codant pour B-Glyc-KDEL.

### I - 2) Purification de protéines

La purification des fragments recombinants est faite également selon la technique décrite dans Su et al, 1991, cité plus haut. Brièvement, des cellules de E. coli contenant des plasmides d'expression recombinants obtenus à partir de pSU108 sont mis en cultures toute la nuit à 30°C. La culture a ensuite été diluée 5 fois dans du LB supplémenté avec 50 mg/ml d'ampicilline à 50°C. Après incubation pendant 4 heures à 42°C, les cellules sont lavées largement dans 10 mM Tris/HCI, pH 8, incubées pendant 10 min. dans 10 mM Tris/HCl, pH8; 25% de sucrose, 1 mM EDTA, et finalement resuspendu rapidement dans un mélange eau-glace contenant 1 mM de PMSF et un mélange d'inhibiteur de protéase (leupeptine, chymostatine, pepstatine, antipaine, et aprotinine). La dernière étape conduit à la rupture du periplasme. Après clarification, le surnageant est chargé sur une colonne QFF (Pharmacia) et élué par un gradient linéaire de NaCl dans 20 mM Tris/HCI, pH 7.5. Selon la construction, le fragment B est élué entre 120 mM et 400 mM. Les fractions contenant le fragment B sont ensuite dialysées contre 20 mM de Tris/HCl, pH 7.5, et rechargées sur une colonne monoQ (Pharmacia) et éluées de la même façon que précédemment. Les protéines résultantes, estimées à un degré de pureté de 95% par gel d'électrophorèse en polyacrylamide-SDS, sont ensuite stockées à -80°C jusqu'à utilisation.

### I - 3) Induction d'une réponse CTL in vitro.

Des cellules dendritiques (CD) sont mises en culture suivant des protocoles préétablis (Romani et al., 1994). En bref, des PBMC sont remises en suspension dans le milieu d'Iscove et incubées pendant 2 h à 37°C dans des boîtes à 6 puits. Les cellules qui n'ont pas adhéré sont enlevées et les cellules restantes sont incubées à 37°C en présence de GM-CSF (800 U/ml) et d'IL-4 (500 U/ml). Après 5 jours de culture, IL-1α et IFN-γ respectivement à une concentration de 50 U/ml et 150 u/ml, sont ajoutés et l'incubation est poursuivie à 30°C pendant 24 h. Les cellules dendritiques sont ensuite remises en suspension dans le milieu d'Iscove en présence de concentrations croissantes de fragment B couplé à l'épitope MAGE et en présence de la β2-microglobuline humaine à 3 µg/ml, ceci afin d'améliorer la capacité des cellules à la présentation des épitopes membranaires. Ce mélange est incubé à 30°C pendant 4 h. Des CD qui ont internalisé le fragment B couplé à cet épitope sont irradiées à 5000 rad, assemblées par centrifugation, remises en suspension, et mélangées à des lymphocytes CD8⁺ (préparées à partir des PBMC). Ces CD pulsées avec un antigène et les CD8⁺ sont ensuite gardées en co-culture en présence d'IL-7 à 5 ng/ml.

Après 10 jours, les lymphocytes CD8⁺ répondeurs sont restimulés par des CD irradiées fraîchement préparées, qui ont été elles aussi incubées en présence de concentrations croissantes de fragment B couplé à ce même épitope. La co-culture de CD et de lymphocytes CD8⁺ répondeurs est poursuivie en présence d'IL-2 et d'IL-7 à 10 U/ml et 5 ng/ml, respectivement. Ce protocole de restimulation est répété 3 fois.

Pour mesurer l'induction d'une réponse de CTL, les lymphocytes CD8⁺ répondeurs pré-stimulés comme décrit ci-dessus sont incubés en présence de cellules cancéreuses ou de cellules infectées par un virus. Ces cellules qui expriment l'épitope choisi ont été marquées avec du Na₂ ⁵¹CrO₄ puis mises en contact avec les CD8⁺ répondeurs pendant 5 h (Bakker et al., 1994). La radioactivité libérée dans le milieu est ensuite déterminée, permettant de quantifier l'activité cytotoxique des lymphocytes CD8⁺ répondeurs pré-stimulés.

### Résultats

### II - Présentation de l'antigène MAGE I par des cellules Pena-EBV et dendritiques pulsées par un fragment B de la toxine Shiga porteur de cet antigène.

### II - 1) Etude morphologique du transport intracellulaire d'un fragment B porteur de l'épitope MAGE-1.

Nous avons montré qu'il est possible de fusionner une séquence peptidique à l'extrémité carboxy-terminale du fragment B de la toxine Shiga tout en préservant le routage intracellulaire de cette protéine vers le réticulum endoplasmique (RE). Cette démonstration a été effectuée en construisant des polypeptides chimériques comprenant le fragment B, le site de N-glycosylation et le signal de rétention KDEL. A titre de témoin, le signal de rétention KDELGL a été utilisé qui est la version inactive du peptide KDEL, Misendock et Rothman, 1995, J. Cell. Biol, 129: 309-319. Par des études morphologiques et biochimiques, il a été montré que le fragment B modifié et transporté de la membrane plasmide via des endosomes et l'appareil de Golgi vers le réticulum endoplasmique. Ce transport est inhibé par le BFA (Brefeldine A métabolite fongique) et diminué par le nocodazole (agent dépolymérisant des microtubules).

Ces expériences montrent clairement que le routage intracellulaire de la protéine de fusion vers le réticulum endoplasmique est préservé. Pour évaluer le potentiel du fragment B en tant que vecteur d'épitope pour la vaccination anti-tumorale *in vitro,* l'épitope MAGE-1 a été rajouté au fragment B-Glyc-KDEL dans les conditions expérimentales décrites plus haut. La nouvelle protéine a été nommée B-MAGE-Glyc-KDEL. La protéine B-MAGE-Glyc-KDEL a été couplée au fluorophore DTAF afin de pouvoir suivre son transport intracellulaire par microscopie confocale. Après son internalisation, cette protéine est détectable au niveau de l'appareil de Golgi ainsi qu'au niveau du RE des cellules HeLa, des cellules Pena-EBV (lignée de lymphocytes B immortalisées à l'aide du virus d'Epstein-Barr). Ces résultats confirment les observations originales concernant le transport intracellulaire d'un fragment B modifié à son extrémité carboxy-terminale (décrit plus haut) et permettent d'affirmer que certaines cellules présentatrices de la lignée hématopoïétiques sont capable d'internaliser le fragment B et de transporter la protéine jusqu'au RE.

Nous allons maintenant préciser ces études en mesurant la N-glycosylation de la protéine B-MAGE-Glyc-KDEL. La N-glycosylation est une modification qui se fait spécifiquement au niveau du RE, et nous avons montré précédemment qu'un fragment B porteur d'un site de reconnaissance pour la N-glycosylation est en fait glycosylé si elle est transportée jusqu'au RE.

### II - 2) Etude de la présentation de l'antigène MAGE-1 par des cellules Pena-EBV et dendritiques pulsées par la protéine B-MAGE-Glyc-KDEL.

Afin d'évaluer la capacité du fragment d'agir en tant que vecteur d'épitope, nous nous sommes servi d'un clone de lymphocytes T cytotoxiques (CTL 82/30) reconnaissant spécifiquement l'épitope MAGE-1 associé au CMH de classe I de cellules présentatrices du haplotype HLA-A1. Ces CTL sont gardées en présence de cellules Pena-EBV ou dendritiques pulsées avec la protéine B-MAGE-Glyc-KDEL. Si l'épitope MAGE-1 est présenté par des cellules présentatrices, les CTL seront activées et sécréteront le l'interféron γ (IFNγ), qui est ensuite dosé.

La quantité d'INFγ sécrétée est proportionnelle à l'amplitude de la stimulation des CTL par des cellules présentatrices.

Des cellules présentatrices (Pena-EBV et dendritiques), 20000 cellules par micropuits à fonds ronds) ont été soit fixées 1 h avec du PBS-paragromaldéhyde 4%, soit non fixées. Elles ont ensuite été lavées 2 fois avec de l'OptiMEM avant d'être incubées pour 15 h avec des dilutions de la protéine B-MAGE-Glyc-KDEL. La protéine a été testée à 4 dilutions, en commençant à une concentration de 10 µM final, et diluant de 5 en 5 dans du milieu OptiMEM (milieu sans sérum). Après 15 h, les plaques ont été lavées 2 fois par centrifugation à basse vitesse. Les CTL (CTL 82/30) ont été rajoutées à raison de 5000 CTL par puits dans 100 µl de milieu de culture (ID-HS-AAG + 25 U/ml d'IL2). Comme contrôle positif, des CTL 82/30 ont été gardées en présence de la lignée G43 (lignée de lymphocytes B transfectées par un plasmide d'expression de MAGE-1). Après 24 h d'incubation, les surnageants ont été récoltés pour doser la quantité d'IFNγ produit.

Les résultats obtenus sont représentés dans le Tableau I.

**Tableau I**

| **Type de cellules** | | **Concentration de B-MAGE-Glyc-KDEL** | | | |
|---|---|---|---|---|---|
| | | 10 µM | 2 µM | 0.4 µM | 0.08 µM |
| | | | | | |
| Dendritiques | fixées | 446 ± 293 | 821 ± 64 | 661 ± 18 | 312 ± 181 |
| | non fixées | 1557 ± 404 | 1315 ± 91 | 1231 ± 150 | 1174 ± 478 |
| | | | | | |
| Pena-EBV | fixées | 70 ± 47 | 68 ± 48 | 23 ± 32 | 4 ± 5 |
| | non fixées | 1966 ± 415 | 1960 ± 206 | 1544 ± 42 | 853 ± 116 |

Les résultats sont représentés par unité d'IFNγ produit dans chaque conditions (moyenne ± écart type; n = 3).

Nous constatons que les cellules dendritiques et les cellules Pena-EBV pulsées avec la protéine B-MAGE-Glyc-KDEL sont bien reconnues par les CTL, même à de faibles concentrations de la protéine. Par contre, les cellules dendritiques et les cellules Pena-EBV, qui ont été fixées préalablement, ne sont pas reconnues. Il semble donc qu'il y ait eu endocytose et processing de la protéine B-MAGE-Glyc-KDEL. Ces résultats encourageants seront maintenant étayés par des expériences de vaccination *in vitro.*

### III - Test d'activité anti-tumorale et/ou antivirale in vivo chez la souris.

Les cellules dendritiques de souris sont préparées et marquées avec un antigène dérivé des protéines P21 RAS, P53 ou EP2/NER pour tester l'activité anti-tumorale ou de HBV, EBV ou HPV pour tester l'activité antivirale. Cette préparation de cellules dendritiques est effectuée selon le protocole présenté en 1- 4) ci-dessus.

Ces cellules dendritique sont ensuite introduites chez la souris.

L'effet antiviral ou anti-tumoral est observé par traitement ultérieur de ces souris ainsi greffées par des cellules tumorales ou des virus exprimant cet antigène.

### Conclusion

Les séquences polypeptidiques ou les séquences polynucléotidiques de l'invention peuvent ainsi avantageusement constituer un principe actif d'une composition pharmaceutique destinée à traiter certains cancers ou certaines infections virales ou bactériennes, à partir du moment où un épitope particulier dudit virus ou de ladite cellule cancéreuse aura été intégrée dans la séquence nucléotidique recombinante, conduisant à une synthèse d'un polypeptide chimérique susceptible d'être restreint par le CMH classe 1 et d'être exprimé en surface membranaire des cellules du système immunitaire.

### IV :- Restauration du transport intracellulaire de la protéine mutée CFTR (ΔF508) à l'aide du fragment B de la toxine de Shiga.

La protéine CFTR (cystis fibrosis transmembrane regulator) est un canal chlore de la membrane plasmique. Chez la grande majorité des patients atteints de mucoviscidose, le gène CFTR porte des mutations. La mutation (ΔF508), la plus fréquente observée, affecte le routage intracellulaire de la protéine CFTR. En effet, la protéine mutée CFTR(ΔF508), qui est fonctionnelle en ce qui concerne son activité canal ionique, reste bloquée au niveau du réticulum endoplasmique, au lieu d'être transportée jusqu'à la membrane plasmique. Nous avons introduit dans le réticulum endoplasmique à l'aide du fragment B de la toxine de Shiga, un domaine de la protéine CFTR connu pour être le domaine d'interaction avec la protéine calnexine ("chaperonne" du RE). Ce domaine sera fusionné à l'extrémité carboxy-terminale du fragment B. Nous avons testé que cette protéine chimérique permet de déplacer les chaînes N-glycosylées de la glycoprotéine CFTR (ΔF508) du site d'interaction avec la calnexine, ce qui a pour effet que la protéine CFTR (ΔF508) n'est plus retenue dans le réticulum endoplasmique et peut être transportée jusqu'à la membrane plasmique et fonctionner alors normalement.

Dans un premier temps, nous avons construit une protéine chimérique composée du fragment B et du domaine d'interaction dérivé de la protéine CFTR. Un signal de recyclage (le peptide KDEL) a été ajouté à l'extrémité carboxy-terminale de cette protéine afin d'augmenter sa rétention dans le réticulum endoplasmique. Il a été tout d'abord vérifié que la nouvelle protéine était elle aussi transportée dans le réticulum endoplasmique des cellules cibles. La mobilisation de CFTR (ΔF508) a été étudiée dans des cellules d'une lignée stable de cellules LLCPK1 transfectées par l'ADNc de CFTR (ΔF508). Cette lignée a été établie par Mlle. M. A. Costa de Beauregard et M. D. Louvard (Institut Curie, Paris, CNRS UMR 144). La protéine CFTR (ΔF508) qui est exprimée dans ces cellules a été de plus dotée d'un épitope tag. Il est donc possible de détecter par immunofluorescence l'arrivée de la protéine CFTR (ΔF508) au sein de la membrane plasmique. En l'absence de traitement, la membrane plasmique des cellules LLCPK1 de la lignée est dépourvue de marquage spécifique de CFTR (ΔF508) au sein de la membrane plasmique. Si les résultats de ces expériences pilotes sont prometteurs, nous nous efforcerons de développer cette approche dans une optique de thérapie de la mucoviscidose.

### Conclusion

L'expérience décrite ci-dessus montre que le polypeptide synthétique dans lequel X est constitué du domaine d'interaction entre le protéine CFTR et la calnexine peuvent avantageusement constituer le principe actif d'une composition thérapeutique destiné à traiter la mucoviscidose. En effet, la compétition entre le domaine d'interaction muté existant chez le mutant et le fragment du polypeptide synthétique pour l'interaction avec la calnexine peut permettre de restaurer la sécrétion au niveau des bronches de la protéine mutée.

### Essai de présentation antigénique :

Les cellules présentatrices d'antigène (CMSP, cellules B-EBV, cellules T, cellules dentritiques) ont été incubées dans des microplaques de 96 puits à une densité de 10⁵ cellules par puits et primées (pulsed) à 37°C pendant 4 heures ou 15 heures avec l'antigène dissous dans 100 µl de milieu d'Iscove. A la fin de l'incubation, le milieu a été enlevé et 20.000 cellules CTL ont été ajoutées à chaque puits dans 100 µl de milieu de culture des CTL contenant 25 U/ml d'IL2. Après 24 heures, 50 µl de surnageant ont été collectés et l'interféron γ a été mesuré par un test ELISA (Diaclone). Dans certaines expériences, les cellules ont été fixées avec 1 % paraformaldéhyde pendant 10 min à la température ambiante et lavées extensivement avant transfert dans des microplaques.

## Revendications

1. Séquence chimérique répondant à la formule B-X ou à la formule B-X' dans laquelle B est le fragment B de la toxine de Shiga, tout fragment dérivé du fragment B de Shiga par mutation, délétion ou addition, présentant les mêmes propriétés d'acheminement dans une cellule cible, que le fragment B de Shiga , X' est un polynucleotide codant pour X et X représente une ou plusieurs séquences d'intérêt thérapeutique choisie(s) parmi les peptides, polypeptides, antigènes ou épitopes d'antigènes.

2. Séquence selon la revendication 1 **caractérisée en ce qu'**elle comprend en outre :
- un site de glycosylation, ou
- un signal de rétention dans le réticulum endoplasmique, ou
- un site de sulfatation, ou
- un mélange de ceux-ci.

3. Séquence selon l'une des revendications 1 à 2 **caractérisée en ce que** X est un ou plusieurs épitopes susceptibles d'être présentés par le complexe majeur d'histocompatibilité de classe 1.

4. Séquence selon la revendication 3 **caractérisée en ce que** X est un épitope issu d'un polypeptide ou d'une protéine que l'on souhaite exprimer à la surface de cellules du système immunitaire et notamment dans le groupe formé de protéines de cellules cancéreuses, de protéines de virus, d'oncogènes.

5. Séquence selon la revendication 4 **caractérisée en ce que** X est un épitope MAGE spécifique de cellules de mélanome.

6. Séquence selon les revendications 1 et 2 **caractérisée en ce que** X est un polypeptide susceptible de restaurer ou d'activer une fonction du transport intracellulaire en interagissant avec des protéines de la machinerie cellulaire, notamment en entrant en compétition dans le réticulum endoplasmique avec la forme mutée d'une protéine impliquée dans le transport intracellulaire, ou en supplémentant une fonction déficiente dans ledit transport.

7. Séquence selon la revendication 6 **caractérisée en ce que** X est le domaine d'interaction de la protéine CFTR avec une molécule chaperonne, la calnexine.

8. Construction d'acide nucléique **caractérisée en ce qu'**elle code pour la séquence chimérique B-X selon l'une quelconque des revendications 1 à 7.

9. Séquence selon l'une des revendications 1 à 7 pour utilisation comme agent actif en immunothérapie par présentation antigénique d'épitopes sur des cellules du système immunitaire.

10. Séquence selon la revendication 9 **caractérisée en ce que** les cellules sont des cellules dendritiques ou des macrophages.

11. Séquence selon la revendication 9 ou 10 **caractérisée en ce que** les épitopes sont issus d'antigènes viraux, parasitaires ou bactériens, ou dérivés de protéines spécifiques de cellules cancéreuses, dérivés d'oncogènes ou dérivés de protéines de virus cancérogènes.

12. Séquence selon l'une des revendications 1 à 2 pour utilisation comme principe actif pour la restauration du transit intracellulaire de protéines mutées dans leur site d'attachement à une molécule chaperonne.

13. Séquence selon la revendication 12 dans laquelle la protéine mutée est la protéine CFTR responsable de la mucoviscidose.

14. Composition à visée thérapeutique, **caractérisée en ce qu'**elle comprend comme principe actif une séquence selon l'une des revendications 1 à 7.

15. Composition selon la revendication 14 **caractérisée en ce que** la séquence est une séquence polypeptidique selon la revendication 1.

16. Utilisation d'une séquence selon l'une quelconque des revendications 1 à 7 pour la fabrication d'un médicament permettant de restaurer des déficits du transport intracellulaire.

17. -Utilisation d'une séquence selon l'une quelconque des revendications 1 à 7 pour la fabrication d'un médicament permettant de stimuler les défenses immunitaires de l'organisme vis-à-vis d'infections virales, parasitaires, bactériennes, ou d'antigènes de cellules cancéreuses.

18. Utilisation d'une séquence selon l'une quelconque des revendications 1 à 7 pour la fabrication d'un médicament permettant de diminuer ou supprimer des réactions immunitaires dans les maladies auto-immunes.

## Claims

1. A chimeric sequence with formula B-X or with formula B-X', in which B is the Shiga toxin B fragment or any fragment derived from the Shiga toxin B fragment by mutation, deletion or addition, having the same routing properties in a target cell as the Shiga toxin B fragment, X' is a polynucleotide coding for X and X represents one or more sequences of therapeutic interest selected from peptides, polypeptides, antigens and epitopes of antigens.

2. A sequence according to claim 1, **characterized in that** it further comprises:
• a glycosylation site; or
• a signal for retention in the endoplasmic reticulum; or
• a sulphatation site; or
• a mixture thereof.

3. A sequence according to claim 1 or claim 2, **characterized in that** X is one or more epitopes which can be presented by the class I major histocompatibility complex.

4. A sequence according to claim 3, **characterized in that** X is an epitope derived from a polypeptide or a protein the expression of which is desired at the surface of cells of the immune system, in particular from the group formed by proteins of cancer cells, virus proteins or oncogenes.

5. A sequence according to claim 4, **characterized in that** X is a MAGE epitope specific to melanoma cells.

6. A sequence according to claim 1 or claim 2, **characterized in that** X is a polypeptide which can restore or activate an intracellular transport function by interacting with proteins of the cell machinery, in particular by competing in the endoplasmic reticulum with the mutated form of a protein involved in intracellular transport, or by supplementing a function which is deficient in said transport.

7. A sequence according to claim 6, **characterized in that** X is the domain of interaction of the CFTR protein with a chaperone molecule, calnexin.

8. A nucleic acid construction, **characterized in that** it codes for the chimeric sequence B-X according to any one of claims 1 to 7.

9. A sequence according to one of claims 1 to 7, for use as an agent which is active in immunotherapy by antigenic presentation of epitopes on the cells of the immune system.

10. A sequence according to claim 9, **characterized in that** the cells are dendritic cells or macrophages.

11. A sequence according to claim 9 or claim 10, **characterized in that** the epitopes are derived from viral, parasitic or bacterial antigens, or derivatives of specific proteins of cancer cells, derivatives of oncogenes or derivatives of cancerogenic virus proteins.

12. A sequence according to claim 1 or claim 2, for use as an active principle for restoring intracellular transit of proteins mutated at their site of attachment to a chaperone molecule.

13. A sequence according to claim 12, in which the mutated protein is the CFTR protein responsible for cystic fibrosis.

14. A composition for therapeutic use, **characterized in that** it comprises, as the active principle, a sequence according to one of claims 1 to 7.

15. A composition according to claim 14, **characterized in that** the sequence is a polypeptide sequence in accordance with claim 1.

16. Use of a sequence according to any one of claims 1 to 7 for the production of a drug for restoring deficiencies in intracellular transport.

17. Use of a sequence according to any one of claims 1 to 7, for the production of a drug for stimulating the immune defences of the organism towards viral, parasitic or bacterial infections or cancer cell antigens.

18. Use of a sequence according to any one of claims 1 to 7 for the production of a drug for reducing or preventing immune reactions in autoimmune diseases.

## Patentansprüche

1. Chimäre Sequenz, die der Formel B-X oder der Formel B-X' entspricht, in der B das Fragment B des Shiga-Toxins ist oder jedes Fragment, welches von dem Fragment B von Shiga durch Mutation, Deletion oder Addition abgeleitet ist und die gleichen Transporteigenschaften in einer Zielzelle aufweist wie das Fragment B von Shiga, und wobei X' ein Polynucleotid ist, das X codiert, und X eine oder mehrere Sequenzen von therapeutischem Interesse darstellt, die ausgewählt sind aus Peptiden, Polypeptiden, Antigenen oder Antigenepitopen.

2. Sequenz nach Anspruch 1, **dadurch gekennzeichnet, dass** sie des weiteren:
- eine Glycosylierungsstelle, oder
- ein Signal zur Retention im Endoplasmatischen Reticulum, oder
- eine Sulfatationsstelle, oder
- eine Mischung davon
umfasst.

3. Sequenz nach einem der Ansprüche 1 und 2, **dadurch gekennzeichnet, dass** X einem oder mehreren Epitopen entspricht, die dazu in der Lage sind, durch den Haupthistokompatibliätskomplex der Klasse I präsentiert zu werden.

4. Sequenz nach Anspruch 3, **dadurch gekennzeichnet, dass** X ein Epitop ist, das von einem Polypeptid oder einem Protein abstammt, von dem beabsichtigt ist, dass es auf der Oberfläche von Zellen des Immunsystems exprimiert wird, und insbesondere aus der Gruppe gebildet aus den Proteinen von Krebszellen, Proteinen von Viren oder Onkogenen.

5. Sequenz nach Anspruch 4, **dadurch gekennzeichnet, dass** X ein MAGE-Epitop ist, das spezifisch für Melanomzellen ist.

6. Sequenz nach den Ansprüchen 1 und 2, **dadurch gekennzeichnet, dass** X ein Polypeptid ist, das in der Lage ist, eine Funktion des intrazellulären Transports wiederherzustellen oder zu aktivieren durch Interaktion mit Proteinen der zellulären Maschinerie, insbesondere beim Durchtritt in das Endoplasmatischen Reticulum im Wettbewerb mit der mutierten Form eines Proteins, das in den intrazellulären Transport involviert ist, oder eine defiziente Funktion beim Transport zu ergänzen.

7. Sequenz nach Anspruch 6, **dadurch gekennzeichnet, dass** X eine Domaine der Interaktion des CFTR-Proteins mit einem Chaperon-Molekül, dem Calnexin, ist.

8. Nucleinsäurekonstrukt, **dadurch gekennzeichnet, dass** es die chimäre Sequenz B-X nach einem der Ansprüche 1 bis 7 codiert.

9. Sequenz nach einem der Ansprüche 1 bis 7 zur Verwendung als Wirkstoff in der Immuntherapie durch antigene Präsentation von Epitopen durch die Zellen des Immunsystems.

10. Sequenz nach Anspruch 9, **dadurch gekennzeichnet, dass** die Zellen dentritische Zellen oder Makrophagen sind.

11. Sequenz nach Anspruch 9 oder 10, **dadurch gekennzeichnet, dass** die Epitope von viralen, parasitären oder bakteriellen Antigenen abstammen oder von Proteinen, die spezifisch für Krebszellen sind, die von Onkogenen oder von Proteinen kanzerogener Viren abgeleitet sind.

12. Sequenz nach einem der Ansprüche 1 und 2 zur Verwendung als Wirkstoff für die Wiederherstellung des intrazellulären Transports von Proteinen, die in der Stelle zur Bindung an ein Chaperon-Molekül mutiert sind.

13. Sequenz nach Anspruch 12, in der das mutierte Protein das CFTR-Protein ist, das für Mucoviszidose verantwortlich ist.

14. Arzneimittel, **dadurch gekennzeichnet, dass** es als Wirkstoff eine Sequenz nach einem der Ansprüche 1 bis 7 umfasst.

15. Arzneimittel nach Anspruch 14, **dadurch gekennzeichnet, dass** die Sequenz eine Polypeptidsequenz nach Anspruch 1 ist.

16. Verwendung einer Sequenz nach einem der Ansprüche 1 bis 7 zur Herstellung eines Medikaments, das die Wiederherstellung eines Defizits beim intrazellulären Transport erlaubt.

17. Verwendung einer Sequenz nach einem der Ansprüche 1 bis 7 zur Herstellung eines Medikaments, das die Stimulierung der Immunabwehr des Organismus gegenüber viralen, parasitären, bakteriellen Infektionen oder gegen Antigene von Krebszellen erlaubt.

18. Verwendung einer Sequenz nach einem der Ansprüche 1 bis 7 zur Herstellung eines Medikaments, welches das Verringern oder Unterdrücken von Immunreaktionen bei Autoimmunerkrankungen erlaubt.
